Europäisches Patentamt

European Patent Office

Office européen des brevets

(19)

(11) Publication number: **0 071 635**

**B1**

(12) **EUROPEAN PATENT SPECIFICATION**

(45) Date of publication of patent specification: **30.04.86**

(21) Application number: **82900761.6**

(22) Date of filing: **28.01.82**

(86) International application number:
**PCT/US82/00109**

(87) International publication number:
**WO 82/02661 19.08.82 Gazette 82/20**

(51) Int. Cl.⁴: **A 61 K 39/44,** A 61 K 43/00,
A 61 K 39/42, A 61 K 39/395

(54) **IMMUNOASSAY FOR MULTI-DETERMINANT ANTIGENS.**

(30) Priority: **30.01.81 US 230175**

(43) Date of publication of application:
**16.02.83 Bulletin 83/07**

(45) Publication of the grant of the patent:
**30.04.86 Bulletin 86/18**

(84) Designated Contracting States:
**AT BE CH DE FR GB LI LU NL SE**

(56) References cited:
**WO-A-82/00058**
**WO-A-82/01072**
**US-A-4 039 652**
**US-A-4 189 464**
**US-A-4 271 145**

**N.J. of EXPERIMENTAL MEDICINE, VOL 148,
pp 383-392, ISSUED 91 AUG., 1978, W.
GERHARD AND R.G. WEBSTER, ANTIGENIC
DRIFT IN INFLUENZA VIRUSES. SEE p. 384**

(73) Proprietor: **CENTOCOR, INC.
244 Great Valley Parkway
Malvern, PA 19355 (US)**

(73) Proprietor: **THE MASSACHUSETTS GENERAL
HOSPITAL
55 Fruit Street
Boston Massachusetts 02114 (US)**

(72) Inventor: **SCHOEMAKER, Hubert J. P.
495 School House Lane
Devon, PA 19333 (US)**
Inventor: **WESTRICK, Barbara L.
6114 Ensley Drive
Flourtown, PA 19031 (US)**
Inventor: **ZURAWSKI, Vincent R. Jr.
628 Northgate Road
West Chester, PA 19380 (US)**
Inventor: **WANDS, Jack R.
210 Varick Road
Waban, MA 02168 (US)**

(74) Representative: **Holdcroft, James Gerald, Dr.
et al
Graham Watt & Co. Riverhead
Sevenoaks Kent TN13 2BN (GB)**

Courier Press, Leamington Spa, England.

(56) References cited:

N, MOLECULAR IMMUNOLOGY, VOL. 16, pp 101-106. ISSUED FEBRUARY, 1979, M.E. FRANKEL AND W. GERHARD, RAPID DETERMINATION OF BINDING CONSTANTS FOR ANTI-VIRAL ANTIBODIES BY A RADIOIMMUNOASSAY

N, IMMUNO CHEMISTRY, Vol. 9, pp. 341-357. ISSUED 1972. D.M. CROTHERS AND H. METZGER. INFLUENCE OF POLYVALENCY ON THE BINDING PROPERTIED OF ANTIBODIES

N, J. of Immunological Methods, VOL. 31, pp. 365-371 ISSUED 27 DEC, 1979. I. MILLMAN AND M. SMITH., 'ASPRIA': A NEW SENSITIVE ASSAY FOR THE DETECTION OF HEPATITIS B SURFACE ANTIGEN

N, FEDERATION PROCEEDINGS, Vol. 39, P. 1204. ISSUED 13 APRIL, 1980. V.R. ZURAWSKI ET AL. MONOCLONAL ANTIBODIES TO TETANUS TOXIN ET AL. PRODUCED BY CLONES OF MURINE HYDRIDOMAS. SEE ABSTRACT 4922

N,J. OF IMMUNOLOGICAL METHODS, VOL. 42 PP 11-15. ISSUED 16 APRIL, 1981. M. UOTILLA ET AL. TWO-SITE SANDWICH IMMUNO ASSAY WITH MONOCLONAL ANTIBODIES TO HUMAN AFP.

N,J. OF IMMUNOLOGICAL METHODS, VOL. 45, pp 255-273 ISSUED 29 SEP. 1981. W. M. HUNTER, AND PS BUDD IMMUNORADIOMETRIC VERSUS RADIOIMMUNOASSAY

N, NATURE, VOL. 290, pp 501-503, ISSUED 09 APRIL, 1981. D.S. SECHER. IMMUNORADIOMETRIC ASSAY OF HUMAN LEUKOCYTE INTERFERON USING MONOCLONAL ANTIBODY

GASTROENTEROLOGY, vol. 79, no. 5, pt. 2, November 1980 J.R. WANDS et al.: "Immunodiagnosis of Hepatitis B by high affinity Monoclonal Anti-HB Antibodies", page 1063, column 1.

GASTROENTEROLOGY, vol. 79, no. 5, pt. 2, November 1980 J.R. WANDS et al.: "Identification of Epitopes on HBsAG polypeptides by analysis with Monoclonal Anti-HBs Antibodies", page 1063, column 1.

FEDERATION PROCEEDINGS, vol. 39, no. 3, March 1, 1980 D.J. PHILLIPS et al.: "Solidphase fluorometric assay for detecting mouse Monoclonal Antibody reactive with influenza Viruses", page 929, column 1, abstract no. 3487.

GASTROENTEROLOGY, vol. 77, no. 5, 1979 J.R. WANDS et al.: "Production and characterisation of Monoclonal Antibodies to Hepatitis B Surface Antigen (HBsAg) by cellular hybridisation", page A46, column 1.

## Description

Technical field

This invention is in the field of immunology and particularly relates to an immunoassay for the detection of multi-determinant hepatitis viral antigens.

Background art

The desirability of having an immunoassay capable of being employed by hospitals and clinical laboratories for the early detection of viral infections has been widely recognized. The need for such an assay can be illustrated, for example, by acute hepatitis B, one form of the various hepatitis viral infections. Acute hepatitis B causes significant mortality and morbidity and chronic infection with this virus is also associated with hepatocellular carcinoma, chronic active hepatitis and cirrhosis. In view of this need, much research has been devoted to the development of immunoassays capable of detecting viral antigens such as hepatitis B surface antigen (HBsAg), the viral marker for hepatitis B, in the blood stream of patients.

Generally, the immunoassays which have been developed to date can be classified as radioimmunoassays (RIA) or immunoradiometric assays (IRMA). In a RIA, the amount of antigen present in a sample is measured indirectly employing a limited amount of antibody to compete for labeled antigen. In an IRMA, antigen is assayed directly by reacting the antigen with excess labeled antibody.

In one class of IRMA assays, the unknown antigen is insolubilized and reacted with labeled antibody. When the antigen is insolubilized by reaction with solid-phase antibody, the assay is termed a "two-site IRMA", "junction test", or "sandwich assay". Sandwich assays are further classified according to their methodology as forward, reverse or simultaneous sandwich assays.

Heretofore, the most widely used immunoassay for viral hepatitis B has been the forward sandwich assay. In this assay, a patient sample containing HBsAg is initially incubated with a solid-phase immunoadsorbent containing immobilized antibody for HBsAg. Incubation is continued for a sufficient period of time to allow HBsAg in the patient sample to bind to immobilized antibody on the solid-phase immunoadsorbent. After this first incubation, the solid-phase immunoadsorbent is separated from the incubation mixture and washed to remove excess antigen and other interfering substances, such as non-specific binding proteins, which also may be present in the patient sample. The solid-phase immunoadsorbent containing HBsAg bound to immobilized antibody is subsequently incubated for a second time with labeled antibody capable of binding to HBsAg, which is multivalent. After the second incubation, another wash is performed to remove unbound labeled antibody from the solid-phase immunoadsorbent thereby removing non-specifically bound labeled antibody. Labeled antibody bound to the solid phase immunoadsorbent is then detected and the amount of labeled antibody detected serves as a direct measure of the amount of HBsAg present in the original patient sample. Such forward sandwich assays are described in the patent literature, and in particular, in U.S. Patent Nos. 3,867,517 and 4,012,294, issued to Chung-Mei Ling.

Despite their widespread use, forward sandwich assays do have certain inherent disadvantages. For example, the requirement to perform two separate incubations as well as two separate washings means that the assay is relatively time-consuming and requires a number of manipulative operations which must be performed by skilled technicians thereby making the forward sandwich assay relatively costly. In addition, the kinetics in the second incubation are relatively slow since labeled antibody must react in this step with antigen bound to antibody immobilized on the solid-phase immunoadsorbent.

The reverse sandwich immunoassay was developed to overcome some of the disadvantages of the forward sandwich assay. In a reverse sandwich assay, the patient sample is initially incubated with labeled antibody after which the solid-phase immunoadsorbent containing immobilized antibody is added and a second incubation is carried out. Thus, the initial washing step of a forward sandwich assay is not required, although a wash is performed after the second incubation period. The major advantages to the reverse sandwich assay compared to the forward sandwich assay are the elimination of the first wash step and the improvement in kinetics of reaction since the labeled antibody and antigen in the patient serum are initially incubated together. Thus, for a total incubation period less than that required to reach equilibrium, it would be expected that the sensitivity of the reverse assay would be higher than that of the forward assay. A reverse sandwich assay has been described in the patent literature in U.S. Patent No. 4,098,876, issued to Roger N. Piasio et al.

Although reverse sandwich assays have potential advantages over forward sandwich assays, they still require two separate incubation periods and one washing.

Simultaneous sandwich assays, although known, have not found widespread use. In a simultaneous sandwich assay, the patient sample, immunoadsorbent having immobilized antibody thereon, and labeled antibody are incubated simultaneously in one incubation step. Although offering certain potential advantages due to the single incubation and lack of washing steps required, simultaneous sandwich assays have previously been found to be less sensitive and reliable than forward and reverse sandwich assays. This would be expected, of course, since the patient sample often contains many non-specific binding molecules, such as proteins, which are not washed from the assay as they are in the forward sandwich assay.

3

# 0 071 635

Disclosure of the invention

This invention relates to a simultaneous sandwich assay for detecting the presence of multi-determinant hepatitis viral antigens in liquid samples, such as the blood of a patient. As pointed out above, in a simultaneous sandwich assay, the patient sample is incubated simultaneously with both an immunoadsorbent containing immobilized antibody for the antigen and labeled antibody for the antigen. Thereafter, labeled antibody bound to the immunoadsorbent is detected as an indication of the amount of antigen present in the patient sample.

The invention provides an immunoassay for detecting the presence of a multi-determinant hepatitis viral antigen in a liquid sample wherein said sample is simultaneously incubated in the presence of an immunoadsorbent containing immobilized antibody for said antigen and labeled antibody for said antigen, and thereafter detecting the amount of labeled antibody bound to said immunoadsorbent as an indication of the amount of antigen present in said sample; characterised in that said immobilized antibody and said labeled antibody comprise high-affinity IgM monoclonal antibodies directed against a multiply-appearing determinant of said antigen.

The invention also provides an immunoassay for detecting the presence of a multi-determinant hepatitis viral antigen in a liquid sample comprising the steps of

(a) forming an incubation mixture containing:

i) liquid sample;

ii) a solid-phase immunoadsorbent containing immobilized high-affinity IgM monoclonal antibody directed against a multiply-appearing determinant of said antigen; and

iii) labeled, soluble, high-affinity, IgM monoclonal antibody directed against a multiply-appearing determinant of said antigen;

(b) incubating said incubation mixture under conditions for a period of time sufficient for antigen in the liquid sample to bind to both immobilized monoclonal antibody and labeled, soluble, monoclonal antibody;

(c) separating said solid-phase immunoadsorbent from the incubation mixture after said incubation; and

(d) detecting for labeled monoclonal antibody bound to said solid-phase immunoadsorbent as an indication of the amount of said antigen in the liquid sample.

One preferred embodiment of this simultaneous sandwich assay employs high-affinity monoclonal IgM antibody for HBsAg for the immobilized and labeled antibody in a particularly sensitive assay for viral hepatitis B.

The simultaneous sandwich assay described herein allows the realization of the previously recognized potential advantages of a simultaneous sandwich assay. For example, the assay is simpler and requires less steps since there is only one incubation and since no washing steps are required. This reduces the amount of skilled technician time required which, in turn, reduces the cost of performing the assay.

More importantly, however, is ·the surprising sensitivity which can be obtained when monoclonal antibodies are employed in a simultaneous sandwich assay. For example, for total incubation times which are comparable, it has been found that the simultaneous sandwich assay described herein can be more sensitive than either a forward or reverse assay for the detection of HBsAg.

Another unexpected advantage of the assay described herein is the wide range of antigen concentration which can be detected, despite the fact that only one incubation is employed and no washing steps are employed. For example, it has been found that this assay is capable of detecting HBsAg over a greater than ten million-fold dilution range.

An immunoassay using IgM antibody is disclosed in Wands et al., *Gastroenterology 79 (5)*, 2 (1980) but this is a reverse sandwich immunoassay.

The simultaneous sandwich assay described herein is useful for the detection of multi-determinant antigens. By "multi-determinant", it is meant that the antigen contains a plurality of the same antigenic determinant (epitope). Sometimes, such antigens have also been referred to in the literature as "polydeterminant" antigens, but the terminology "multi-determinant" will be used herein to describe such antigens.

Another term used to describe antigenic determinants is the term "multivalent". The term "multivalent" is used herein to mean that the antigen has a plurality of different antigenic determinants or epitopes thereon.

Many viral antigens are both multi-determinant and multivalent. For example, the hepatitis B virus is known to be a complex 42 nanometer (nm) particle having at least three well defined antigens. These include the hepatitis B core antigen (HBcAg), the hepatitis Be antigen (HBeAg), and the hepatitis B surface antigen (HBsAg).

The HBsAg antigen is a high molecular weight complex protein which occurs on the surface of the intact hepatitis B virus. However, HBsAg is also present in the blood of patients infected with hepatitis B as a circulating 22 nm particle or filamentous form. Because of its presence in the bloodstream of infected patients, HBsAg has become the most widely employed specific viral marker for acute and chronic hepatitis B infection.

HBsAg has been found in patient serum samples at concentrations of up to about 800 micrograms/ml.

4

Data indicate, however, that serum concentrations as low as about 50 pg/ml can be infectious. This represents an extraordinarily wide range for an assay to cover.

The antigens are assayed in a liquid sample. Often, the liquid sample is a sample of a patient's blood or a blood component such as plasma or serum. Other liquid samples, such as urine, could of course be assayed by the simultaneous sandwich assay described herein.

In this assay, high-affinity IgM monoclonal antibodies are employed for both immobilized antibody bound to an immunoadsorbent as well as for labeled antibody.

The term "high-affinity" is employed herein to mean that the antibody has an affinity binding constant (K) greater than $10^9 n^{-1}$, and preferably greater than $10^{11} n^{-1}$. Binding constants are determined from Scatchard plots employing known techniques, and are, of course, determined for the whole antigen and antibody molecules rather than on a per site basis.

The term "monoclonal" means that the antibody has been produced by a cell line cloned from a single antibody producing cell. Monoclonal antibodies are extraordinarily pure, uniform and reproduceable since each antibody is effective against a single antigenic determinant.

Monoclonal antibodies can be obtained in significant quantities from hybridoma cells. Hybridoma cells are fused cells resulting from the fusion of antibody producing cells with tumor cells. The initial work relating to the production of such hybridoma cells was done by Cesar Milstein and George Köhler employing mouse myeloma cells with spleen cells taken from mice immunized with sheep red blood cells. See, Kohler et al., *Eur. J. Immunol., 6*, 511—19 (1976); Köhler et al., *Nature, 256,* 495—7 (1975); and Milstein, *Scientific American, 243 (4),* 66—74 (1980).

More recently, Hilary Koprowski and collegues have extended the original hybridoma work by developing hybridoma cell lines capable of producing monoclonal antibodies against specific viruses and tumors. See, U.S. Patent Nos. 4,196,265 and 4,172,124, respectively. Even more recently, the hybridoma technology has been further extended to produce hybridoma cell lines capable of producing monoclonal antibodies to hepatitis virus. See U.S. application Serial No. 86,947, US Patent No. 4271145 filed October 22, 1979 in the names of Jack R. Wands and Vincent R. Zurawski. It has also been recently discovered that monoclonal IgM antibodies produced from hybridoma cell lines can be employed in certain immunoassays to improve the sensitivity and specificity thereof. See, U.S. application Serial No. 188,735, Int. Pub. No. WO 82/01072, filed September 19, 1980, in the names of Jack R. Wands, Vincent R. Zurawski, and Hubert J. P. Schoemaker.

Thus, the general scientific and patent literature include many descriptions of hybridoma cell lines and monoclonal antibodies produced from such cell lines. These teachings are relied on in general herein, and specifically the teachings of each of the heretofore mentioned articles and patents relating to hybridoma cel lines and/or monoclonal antibodies are hereby incorporated by reference. Additionally, a clone of cell line H25D3, identified in these prior applications, has been deposited at the American Type Culture Collection (ATCC), Rockville, Md., and is identified by ATCC Accession No. HB8058.

As mentioned above, high-affinity IgM monoclonal antibodies for the multi-determinant antigen being assayed are immobilized to a solid-phase immunoadsorbent in the simultaneous sandwich assay of this invention. There are many such solid-phase immunoadsorbents which have been employed. Well-known immunoadsorbents include beads formed from glass polystyrene, polypropylene, dextran, and other materials; tubes formed from or coated with such materials; etc. The antibody can be either covalently or physically bound to the solid-phase immunoadsorbent by techniques such as covalent bonding via an amide or ester linkage or adsorption. Those skilled in the art will know many other suitable solid-phase immunoadsorbents and methods for immobilized antibodies thereon, or will be able to ascertain such using no more than routine experimentation.

High-affinity IgM monoclonal antibody is also used as the labeled antibody in the simultaneous sandwich assay described herein. Such antibodies can be labeled with a radioactive material, such as $^{125}$I; labeled with an optical label, such as a fluorescent material; labeled with an enzyme; or labeled by some other known technique.

The liquid sample, solid-phase immunoadsorbent with immobilized monoclonal antibody, and labeled soluble monoclonal antibody are incubated under conditions and for a period of time sufficient to allow antigen to bind to both the immobilized antibody and the labeled antibody. In general, it is usually desirable to provide incubation conditions sufficient to bind as much antigen as possible because this maximizes the binding of labeled antibody to the solid phase thereby increasing the signal. Typical conditions of time and temperature are two hours at 45°C or twelve hours at 37°C.

Labeled antibody typically binds to antigen more rapidly than immobilized antibody since the former is in solution whereas the latter is bound to a solid-phase support. Because of this, labeled antibody should be employed in a lower concentration than immobilized antibody and it is also preferable to employ a high specific activity for labeled antibody. For example, labeled antibody might be employed at a concentration of about 1—50 ng/assay whereas immobilized antibody might have a concentration of 100—500 ng/assay. The labeled antibody might have a specific activity with, for instance, one iodine per IgM or as high as two labeled iodines per molecule of IgM antibody.

Of course, the specific concentrations of labeled and immobilized antibody, the temperature and time of incubation as well as other such assay conditions will depend upon many factors, including the specific antigen being assayed, the concentration of the antigen, and the monoclonal antibody or antibodies

5

employed. Those skilled in the art will be able to determine operative and optimal assay conditions for each determination desired by employing routine experimentation.

After the single incubation period, the solid-phase immunoadsorbent is removed from the incubation mixture. This can be accomplished by any of the known separation techniques, such as sedimentation or centrifugation. A washing step is not required prior to detection of bound labeled antibody. Detection can be performed by a scintillation counter, for example, if the label is a radioactive gamma emitter, or by a fluorometer, for example, if the label is a fluorescent material. In the case of an enzyme label, the detection can be done by colorimetric methods employing a competing substrate for the enzyme.

As noted above, results which have been achieved employing a simultaneous sandwich assay as described herein have demonstrated that such an assay can be more sensitive than forward and reverse sandwich assays wherein the total incubation time for each assay is the same. This is surprising, and the reason for such sensitivity is not well understood. While not wishing to be bound by this theory, one possible explanation is that the monoclonal IgM antibodies employed tend to form a crosslinked structure in the incubation mixture. Because of this, greater amounts of antigen and labeled antibody can be bound to the solid-phase immunoadsorbent. Data indicate, indirectly, for example, that more antigen molecules can bind to the solid phase than would be expected from the number of antibody molecules thus supporting the concept of crosslinking or layering.

The simultaneous sandwich assay of this invention will now be more specifically illustrated by way of the following examples.

Example 1
Immunoradiometric assay for HBsAG
Forward, reverse and simultaneous sandwich assays were compared for detection of HBsAg.

The monoclonal antibody employed in each was a monoclonal IgM antibody having high affinity for HBsAg and produced by the hybridoma cell line 5D3. The 5D3 hybridoma cell line comprises a cloned line developed by fusing the myeloma cell line P3-NS1/1-Ag4-1 derived from a BALB/c mouse MOPC 21 myeloma with mouse spleen cells immunized by a specific immunization procedure with HBsAg. The specific procedure employed is detailed in copending application U.S. Serial No. 86,947, US Patent 4271145 filed October 22, 1979, the teachings of which are hereby incorporated by reference.

The solid-phase immunoadsorbent was provided by 1/4 inch (6.35 mm) polystyrene beads obtained from Precision Plastic Ball Company, Chicago, Illinois. The beads were coated with the monoclonal IgM antibody as follows. Ascites fluid derived from an immunized BALB/c mouse was diluted 1:500 to 1:5,000 in phosphate buffered saline (PBS). The beads were incubated for 16 hours at room temperature and washed three times with distilled water.

Monoclonal IgM antibody was labeled using the Bolten Hunter method. Specific activity ranged from about 2 to about 20 µCi/µg.

The forward sandwich assay protocol involved incubation of the beads having immobilized monoclonal IgM antibody thereon with 100 µl 1% bovine albumin in PBS and 100 µl of an HBsAg-containing patient serum for one hour at 45°C followed by washing three times with distilled water and subsequent addition of 200 µl of labeled antibody which was incubated for one hour at 45°C.

In the reverse sandwich assay protocol, 100 µl of labeled antibody were incubated with 100 µl of HBsAg-containing patient serum in a soluble phase for 1 hour at 45°C. Subsequently, monoclonal antibody coated onto the solid beads was added for an additional 1 hour at 45°C. The beads were subsequently washed three times with distilled water and placed in a scintillation counter.

The simultaneous sandwich assay protocol involved simultaneously incubating coated beads and 100 µl of labeled antibody and 100 µl of patient sample for two hours at 45°C. The beads were subsequently washed three times with distilled water and placed in a scintillation counter.

Additionally, patient samples were diluted a number of times by a factor of 10 and reassayed by each protocol to determine the limit of sensitivity for each assay protocol. A negative control consisting of human serum was employed.

The results are presented in Table I. As can be seen from the data in Table I, the simultaneous sandwich assay was more sensitive than either the forward or reverse assays where each assay employed the same monoclonal IgM antibody against HBsAg and wherein incubation conditions for each assay were the same.

TABLE I

Comparison of reverse, forward and simultaneous sandwich assays using a radioimmuno assay

| | Forward | | Reverse | | Simultaneous | |
|---|---|---|---|---|---|---|
| | CPM | S/N | CPM | S/N | CPM | S/N |
| Negative control | 407.2 | — | 149.6 | — | 63.5 | — |
| Patient sample | 28,951 | 71.1 | 2,059 | 13.8 | 1,760 | 27.7 |
| 1:10 | 38,474 | 94.5 | 8,017 | 54.4 | 9,246 | 145.1 |
| 1:100 | 38,100 | 93.6 | 12,673 | 84.5 | 16,650 | 262.2 |
| 1:1000 | 11,892 | 29.2 | 8,091 | 53.9 | 8,725 | 137.4 |
| 1:10,000 | 2,921 | 7.2 | 1,840 | 12.3 | 2,311 | 36.4 |
| 1:100,000 | 799 | 1.9 | 361 | 2.4 | 1,024 | 16.1 |
| 1:1,000,000 | 546 | 1.3 | 99 | 0.66 | 582 | 9.2 |

Notes
1. Data is expressed in Counts Per Minute (cpm's).
2. In all assays, identical number of total counts were added and identical reagents were used.
3. S/N in ratio of cpm in sample divided by cpm is negative control. A S/N of greater than 2.0 represents a positive.

Example 2
Clinical patient study

A comparative clinical patient study was made employing 290 patient samples collected at Massachusetts General Hospital from different sources. The simultaneous sandwich assay employed is the one described in Example 1. Commercial assays employed are noted. The results were:

TABLE II

| Patient group | (1)<br>No. Pos.<br>SSA | (2)<br>No. Pos.<br>Comm. | (3)<br>No. Pos.<br>anti-HBc | (4)<br>No. Pos.<br>anti-HBs | Total No.<br>studied |
|---|---|---|---|---|---|
| Acute hepatitis B | 42 | 42 | 36 | — | 42 |
| Acute hepatitis | 12 | 0 | 6 | 6 | 22 |
| Post-transfusion hepatitis | 9 | 0 | 6 | 3 | 25 |
| Blood donors | 5 | 1 | 3 | 0 | 92 |
| Alcoholic liver disease | 2 | 0 | 2 | 0 | 50 |
| Normal controls | 0 | 0 | 6 | 7 | 59 |
| | | | | Total | 290 |

(1) Simultaneous sandwich assay
(2) Abbott Ausria II assay
(3) Abbott Corab assay
(4) Abbott Ausab assay
(5) Anti-HBc titer>1:1024 on 2 positives in SSA

It should be noted that the SSA detected positive samples in cases of acute hepatitis and post-transfusion hepatitis whereas the commercial assay produced negative results.

7

**0 071 635**

Industrial applicability

The invention described herein is useful in the detection of multi-determinant hepatitis viral antigens, such as hepatitis B surface antigen (HBsAg). Thus, the assay can be employed by hospitals or clinical laboratories for the early detection of hepatitis viral infection or the presence of non-viral antigens.

**Claims**

1. An immunoassay for detecting the presence of a multi-determinant hepatitis viral antigen in a liquid sample wherein said sample is simultaneously incubated in the presence of an immunoadsorbent containing immobilized antibody for said antigen and labeled antibody for said antigen, and thereafter detecting the amount of labeled antibody bound to said immunoadsorbent as an indication of the amount of antigen present in said sample, characterised in that said immobilized antibody and said labeled antibody comprise high-affinity IgM monoclonal antibodies directed against a multiply-appearing determinant of said antigen.

2. An immunoassay for detecting the presence of a multi-determinant hepatitis viral antigen in a liquid sample comprising the steps of
   (a) forming an incubation mixture containing:
   i) liquid sample;
   ii) a solid-phase immunoadsorbent containing immobilized high-affinity IgM monoclonal antibody directed against a multiply-appearing determinant of said antigen; and
   iii) labeled, soluble high-affinity, IgM monoclonal antibody directed against a multiply-appearing determinant of said antigen;
   (b) incubating said incubation mixture under conditions and for a period of time sufficient for antigen in the liquid sample to bind to both immobilized monoclonal antibody and labeled, soluble, monoclonal antibody;
   (c) separating said solid-phase immunoadsorbent from the incubation mixture after said incubation; and
   (d) detecting for labeled monoclonal antibody bound to said solid-phase immunoadsorbent as an indication of the amount of said antigen in the liquid sample.

3. An immunoassay according to claim 1 or claim 2, wherein said antigen comprises a virus or antigenically-active portion thereof.

4. An immunoassay according to any one of claims 1, 2 and 3, wherein said hepatitis viral antigen is HBsAg.

5. An immunoassay according to any one of the preceding claims, wherein said labeled, monoclonal antibody and said immobilized monoclonal antibody comprise the same high affinity IgM monoclonal antibody.

6. An immunoassay according to any one of the preceding claims, wherein said labeled, monoclonal antibody contains a radioactive label.

7. An immunoassay according to claim 6, wherein said radioactive label comprises [125]I.

8. An immunoassay according to any one of claims 1 to 5, wherein said labeled, monoclonal antibody is labeled with an enzyme label.

9. An immunoassay according to any one of claims 1 to 5, wherein said labeled, monoclonal antibody is labeled with an optical label.

10. An immunoassay according to claim 9, wherein said optical label comprises a fluorescent material.

**Patentansprüche**

1. Immunotestverfahren zur Feststellung des Vorhandenseins eines multibestimmenden Hepatitisvirusantigens in einer Flüssigprobe, bei dem die Probe gleichzeitig in Gegenwart eines einen immobilisierten Antikörper für das Antigen enthaltenden Immunoadsorbens und eines Indiakatorantikörpers für das Antigen einem Inkubationsprozeß unterzogen und danach die Menge des an das Immunoadsorbens gebundenen Indikatorantikörpers als Anzeige für die in der Probe vorhandene Antigenmenge festgestellt wird, dadurch gekennzeichnet, daß der immobilisierte Antikörper und der Indikatorantikörper Igm-Monoklonal-Antikörper hoher Affinität umfassen, die gegen eine mehrfach-erscheinende Bestimmende des Antigens gerichtet sind.

2. Immunotestverfahren zur Feststellung des Vorhandenseins eines multibestimmenden Hepatitisvirusantigens in einer Flüssigprobe, bestehend aus folgenden Schritten:
   (a) Es wird ein Inkubationsgemisch gebildet aus:
   i) einer Flüssigprobe,
   ii) einem Flüssigphasen-Immunoadsorbens, das einen gegen eine mehrfach-erscheinende Bestimmende des Antigens gerichteten immobilisierten IgM-Monoklonal-Antikörper hoher Affinität enthält, und
   iii) einem gegen eine mehrfach-erscheinende Bestimmende des Antigens gerichteten, lösbaren Indikator-IgM-Monoklonal-Antikörper hoher Affinität;
   b) das Inkubationsgemisch wird unter solchen Bedingungen und für einen solchen Zeitraum einem

8

Inkubationsprozeß unterzogen, daß das Antigen in der Flüssigprobe sowohl eine Verbindung mit dem immobilisierten Monoklonal-Antikörper als auch mit dem lösbaren Indikator-Monoklonal-Antikörper eingeht;

c) das Festphasen-Immunoadsorbens wird von dem Inkubationsgemisch nach der Inkubation getrennt und

d) der an das Flüssigphasen-Immunoadsorbens gebundene Indikator-Monoklonal-Antikörper wird als Anzeige der Antigenmenge in der Flüssigprobe festegestellt.

3. Verfahren nach Anspruch 1 oder 2, dadurch gekennzeichnet, daß das Antigen einen Virus oder einen antigenaktiven Bereich desselben umfaßt.

4. Verfahren nach einem der Ansprüche 1 bis 3, dadurch gekennzeichnet, daß das Hepatitisvirusantigen HBsAg ist.

5. Verfahren nach einen der vorhergehenden Ansprüche, dadurch gekennzeichnet, daß der Indikator-Monoklonal-Antikörper und der immobilisierte Monoklonal-Antikörper den gleichen Igm-Monoklonal-Antikörper hoher Affinität umfassen.

6. Verfahren nach einem der vorhergehenden Ansprüche, dadurch gekennzeichnet, daß der Indikator-Monoklonal-Antikörper einen radioaktiven Indikator enthält.

7. Verfahren nach Anspruch 6, dadurch gekennzeichnet, daß der radioaktive Indikator $^{125}$I umfaßt.

8. Verfahren nach einem der Ansprüche 1 bis 5, dadurch gekennzeichnet, daß der Indikator-Monoklonal-Antikörper mit einem Enzym-Indikator gekennzeichnet ist.

9. Verfahren nach einem der Ansprüche 1 bis 5, dadurch gekennzeichnet, daß der Indikator-Monoklonal-Antikörper mit einem optischen Indikator gekennzeichnet ist.

10. Verfahren nach Anspruch 9, dadurch gekennzeichnet, daß der optische Indikator aus fluoreszierendem Material besteht.

## Revendications

1. Essai immunologique pour détecter la présence d'un antigène multi-déterminant de l'hépatite virale dans und échantillon liquide dans lequel cet échantillon est simultanément incubé en présence d'un immunoadsorbant contenant de l'anticorps immobilisé de cet antigène et de l'anticorps marqué de ce antigène, et ensuite détecter la quantité d'anticorps marqué lié à cet immunoadsorbant comme une indication de la quantité d'antigène présent dans cet échantillon, caractérisé en ce que cet anticorps immobilisé et cet anticorps marqué comprennent des anticorps monoclonaux IgM de haute affinité dirigés contre un multi-déterminant de cet antigène.

2. Essai immunologique pour détecter la présence d'un antigène multi-déterminant de l'hépatite virale dans un échantillon liquide comprenant les étapes de

(a) former un mélange d'incubation contenant:
  i) échantillon liquide;
  ii) un immunoadsorbant à phase solide contenant un anticorps monoclonal IgM de haute affinité immobilisé dirigé contre un multi-déterminant de ce antigène; et
  iii) un anticorps monoclonal IgM de haute affinité soluble, marqué, dirigé contre un multi-déterminant de cet antigène;
(b) incuber ce mélange d'incubation sous des conditions et pendant une période de temps suffisante pour que l'antigène de l'échantillon liquide se lie à la fois à l'anticorps monoclonal immobilisé et à l'anticorps monoclonal, soluble, marqué;
(c) séparer cet immunoadsorbant à phase solide du mélange d'incubation après cette incubation; et
(d) détecter l'anticorps monoclonal, marqué lié à cet immunoadsorbant à phase solide comme une indication de la quantité de cet antigène dans l'échantillon liquide.

3. Essai immunologique selon la revendication 1 ou 2 dans lequel cet antigène comprend un virus ou une partie antigéniquement active de celui-ci.

4. Essai immunologique selon l'une quelconque des revendications 1, 2 et 3 dans lequel l'antigène de l'hépatite virale est HBsAg.

5. Essai immunologique selon l'une quelconque des revendications précédentes dans lequel cet anticorps monoclonal, marqué et cet anticorps monoclonal immobilisé comprennent le même anticorps monoclonal IgM de haute affinité.

6. Essai immunologique selon l'une quelconque des revendications précédentes dans lequel cet anticorps monoclonal, marqué contient un marqueur radio-actif.

7. Essai immunologique selon la revendication 6, dans lequel le marqueur comprend $^{125}$I.

8. Essai immunologique selon l'une quelconque des revendications 1 à 5, dans lequel cet anticorps monoclonal marqué, est marqué par un marqueur à enzyme.

9. Essai immunologique selon l'une quelconque des revendications 1 à 5, dans lequel cet anticorps monoclonal marqué, est marqué par un marqueur optique.

10. Essai immunologique selon la revendication 9, dans lequel ce marqueur optique comprend une matière fluorescente.